**Europäisches Patentamt**

**European Patent Office**

**Office européen des brevets**

(11) Veröffentlichungsnummer: **0 640 827 A1**

## (12) EUROPÄISCHE PATENTANMELDUNG

(21) Anmeldenummer: **94112905.8**

(22) Anmeldetag: **19.08.94**

(51) Int. Cl.6: **G01N 21/35**, G01N 33/32

(30) Priorität: **24.08.93 DE 4328357**

(43) Veröffentlichungstag der Anmeldung: **01.03.95 Patentblatt 95/09**

(84) Benannte Vertragsstaaten: **AT CH DE ES FR GB IT LI NL PT SE**

(71) Anmelder: **Herberts Gesellschaft mit beschränkter Haftung Christbusch 25 D-42285 Wuppertal (DE)**

(72) Erfinder: **Rankl, Franz-Josef, Dr. Roeberstrasse 40 D-42110 Wuppertal (DE)**
Erfinder: **Hartmut, Thomas, Dr. Rheingoldstrasse 1a D-42859 Remscheid (DE)**
Erfinder: **Basener, Norbert Giradestrasse 36 D-42109 Wuppertal (DE)**
Erfinder: **Bonn, Günther K., Prof.Dr. Bahnhof Umgebung 3/6k A-6170 Zirl (AT)**
Erfinder: **Klampfl, Christian, Dr. Dr. Stumpfstrasse 3 A-6020 Innsbruck (AT)**

(74) Vertreter: **Türk, Gille, Hrabal, Leifert Brucknerstrasse 20 D-40593 Düsseldorf (DE)**

(54) **Verfahren zur quantitativen Erfassung der nichtflüchtigen von Pigmenten und Füllstoffen unterschiedlichen Komponenten von Überzugsmitteln oder Überzugsmittelbestandteilen.**

(57) Beschrieben wird ein Verfahren zur quantitativen Erfassung der nichtflüchtigen, von Pigmenten und Füllstoffen unterschiedlichen Komponenten von Überzugsmitteln oder Überzugsmittelbestandteilen. Das Verfahren ist beispielsweise für die Produktkontrolle oder für die Wiederaufbereitung von Überzugsmitteln im Recyclingverfahren geeignet. Das Überzugsmittel oder der Überzugsmittelbestandteil wird in flüssiger, pigmentfreier Form auf eine für die Infrarot-Spektroskopie geeignete transparente Unterlage aufgebracht und auf dieser wird unter zumindest teilweiser Entfernung der flüchtigen Anteile ein homogener Film gebildet. Von diesem Film wird ein Infrarot-Spektrum erstellt und aus diesem wird die Menge der interessierenden Komponenten an Hand der Partial Least Squares Methode unter Auswertung der Infrarot-Spektren von unter identischen Bedingungen aus Überzugsmitteln bekannter quantitativer Zusammensetzung mit unterschiedlichen Konzentrationen der Komponenten erstellten Filmen, berechnet.

EP 0 640 827 A1

Die Erfindung betrifft ein Verfahren zur quantitativen Erfassung der nichtflüchtigen, von Pigmenten und Füllstoffen unterschiedlichen Komponenten von Überzugsmitteln oder Überzugsmittelbestandteilen. Die nichtflüchtigen Komponenten, wie filmbildende Bindemittel, Vernetzer und teilweise auch Additive lassen sich quantitativ genau erfassen.

Die quantitative Erfassung der einzelnen Komponenten von Überzugsmitteln und Überzugsmittelbestandteilen ist bei der Produktkontrolle von Bedeutung. In den letzten Jahren ist das Recycling von Lackresten, Overspray (Lacknebel), Ultrafiltrat von Lacken, Lackkoagulat und von Fehlansätzen bei der Lackherstellung aus wirtschaftlichen Gründen und zur Vermeidung von Umweltbelastungen wichtig geworden. Auch hierfür ist eine genaue quantitative Erfassung der Einzelkomponenten dieser Materialien unerläßlich.

Bisher sind verläßliche Methoden zur quantitativen Bestimmung flüchtiger Komponenten und von Pigmenten und Füllstoffen aus Überzugsmitteln bekannt. Beispielsweise kann die Abtrennung von Pigmenten und Füllstoffen von Bindemitteln, Vernetzungsmitteln und Additiven durch Zentrifugieren erfolgen.

Geeignete Methoden zur quantitativen Bestimmung der nichtflüchtigen, von Pigmenten und Füllstoffen unterschiedlichen Komponenten, wie filmbildenden Bindemitteln, Vernetzungsmitteln und verschiedenen Additiven sind jedoch bisher nicht bekannt. Der Versuch Fällungsanalysen unter Ausnutzung der unterschiedlichen Löslichkeiten der Komponenten durchzuführen erwies sich als unbrauchbar. Auch die Flüssigchromatographie hat sich zur quantitativen Erfassung der nichtflüchtigen, pigmentfreien Komponenten von Überzugsmitteln als nur teilweise praktizierbar erwiesen, beispielsweise für die quantitative Bestimmung spezieller Additive. Darstellungen der Flüssigchromatographie finden sich bei L.R. Synder und J.J. Kikeland in "Introduction to Modern Liquid Chromatography", John Wiley & Sons, New York, 2. Auflage, 1979, bei V. Meyer in "Praxis der Hochleistungs-Flüssigchromatographie", Verlag Diesterweg, Salle, Verlag Sauerländer, Frankfurt/M., 1990 und bei H. Engelhardt in "Practise of High-Performance Liquid Chromatography", Springer Verlag, Heidelberg, 1986.

Mit Hilfe der Veröffentlichungen von J.T. Vandenberg in "An Infrared Spectroskopy Atlas for the Coating Industrie", Philadelphia 1980 und von D. Hummel in "Atlas der Polymer- und Kunststoffanalyse" sind nur qualitative Analysen möglich, jedoch nicht die quantitative Erfassung der nichtflüchtigen, pigmentfreien Komponenten von Überzugsmitteln.

Aufgabe der Erfindung ist daher die Bereitstellung eines Analysenverfahrens zur quantitativen Bestimmung der nichtflüchtigen, von Pigmenten und Füllstoffen unterschiedlichen Komponenten von Überzugsmitteln, bei dem Resultate mit einer Genauigkeit erhalten werden, die beispielsweise für die Produktkontrolle und für umweltfreundliche Verfahren, wie das Recycling von Lackresten oder Overspray und für das Aufarbeiten bzw. die Wiederaufbereitung von Koagulaten und Fehlansätzen, notwendig sind.

Es hat sich gezeigt, daß diese Aufgabe gelöst werden kann durch das den Gegenstand der Erfindung bildende Verfahren zur quantitativen Erfassung der nichtflüchtigen, von Pigmenten und Füllstoffen unterschiedlichen Komponenten von Überzugsmitteln oder Überzugsmittelbestandteilen, das dadurch gekennzeichnet ist, daß man die Überzugsmittel oder Überzugsmittelbestandteile in flüssiger, pigmentfreier Form auf eine für die Infrarot-Spektroskopie geeignete transparente Unterlage aufbringt und auf dieser, unter zumindest teilweiser Entfernung der flüchtigen Anteile, einen homogenen Film bildet, von diesem Film ein Infrarot-Spektrum erstellt und aus diesem die Menge der interessierenden Komponenten an Hand der Partial Least Squares Methode, unter Auswertung der Infrarot-Spektren von unter identischen Bedingungen aus Überzugsmitteln bekannter quantitativer Zusammensetzung mit unterschiedlichen Konzentrationen der Komponenten, erstellten Filmen, berechnet.

Soll ein Überzugsmittel oder ein Überzugsmittelbestandteil (im folgenden auch als "Probe" bezeichnet) quantitativ bestimmt werden, das Pigmente und/oder Füllstoffe (Extender Pigments) enthält, so werden diese vor der Erstellung des homogenen Films entfernt, um eine Trennung von den übrigen Komponenten zu erreichen. Dies kann insbesondere durch Zentrifugieren erfolgen. Zum Zentrifugieren kann die Probe beispielsweise verdünnt werden. Unpigmentierte und keine Füllstoffe enthaltende Proben bedürfen dieser Vorbereitung nicht.

In manchen Fällen kann es günstig sein, aus wäßrigen Proben das Wasser zu entfernen und dieses beispielsweise durch organische Lösemittel, wie beispielsweise Tetrahydrofuran und Propanol-2 zu ersetzen. Dies ist besonders dann günstig, wenn als transparente Unterlage für die Infrarot-Spektroskopie ein wasserlösliches Material, wie ein Kaliumbromid-Preßling verwendet werden soll. Das Wasser kann durch Destillation, beispielsweise durch azeotrope Destillation entfernt werden. Beispielsweise kann die obere Phase eines zentrifugierten Materials mit einem organischen Lösemittel, wie vorstehend erwähnt, versetzt werden, worauf das Wasser abdestilliert werden kann.

Als transparente Unterlage werden übliche für die Infrarot-Spektroskopie geeignete Materialien verwendet. Beispiele sind Kaliumbromid-Preßlinge oder Zinksulfid-Fenster (Irtran-[R] Fenster). Das von Pigmenten und Füllstoffen freie Überzugsmittel bzw. dessen Bestandteil wird auf die transparente Unterlage aufgebracht, wobei ein Naßfilm erzielt wird. Um einen Naßfilm mit definierter Schichtdicke zu erzielen, kann es beispielsweise günstig sein die Unterlage, die mit der Probe bedeckt ist, in üblicher Weise einer horizontalen Drehung mit geeigneter Umdrehungszahl zu unterziehen.

Aus dem Naßfilm wird ein homogener Film gebildet, was unter zumindest teilweiser Entfernung der flüchtigen Anteile erfolgt. Hierzu kann der Naßfilm beispielsweise einer erhöhten Temperatur ausgesetzt werden, was beispielsweise im Trockenofen erfolgen kann. Die Temperatur richtet sich nach der Charakteristik des Überzugsmittels und ist in den meisten Fällen bekannt. Sollte sie nicht bekannt sein, so kann durch Versuche ermittelt werden, bei welcher Temperatur ein homogener, brauchbarer Film entsteht. Die Temperatur, welche zur Abdampfung von Lösemitteln oder zur Vernetzung des Films notwendig ist, liegt im allgemeinen beispielsweise bei 60 bis 180°C. Wenn Lösemittel oder Neutralisationsmittel, wie z.B. Triethanolamin, durch Erwärmen nicht entweicht, muß das Lösemittel oder das Neutralisationsmittel zusätzlich in das Eichprogramm für die Partial Least Squares Methode (PLS-Verfahren) aufgenommen werden.

Die Auswertung der Infrarot-Spektren erfolgt rechnerisch durch die bekannte statistische Partial Least Squares Methode (PLS-Methode), wie sie beispielsweise von Jan-Bernd Lohmüller in seinem Buch "Latent Variable Path Modeling with Partial Least Squares", Physica-Verlag Heidelberg 1989, beschrieben wurde. Diese Methode wurde anfangs entwickelt zur Modellierung einer informationskargen Situation in der Sozialwissenschaft. Sie kann in weiterem Sinn für kausal voraussagende Analysen von komplexen Problemen verwendet werden. In den letzten Jahren hat sie auch in der Chemie Eingang gefunden. Walter Lindberg und Jan Ake Persson berichten beispielsweise über "Partial Least Squares Method for Spectrofluorimetrie Analysis of Mixtures of Humic Acid and Ligninsulfate" in American Chemical Society, 1983, Seiten 643 - 648 und Ildiko E. Frank et al. berichten beispielsweise über "Prediction of Product Quality from Spectral Data Using the Partial Least Squares Method" in der gleichen Zeitschrift 1984, Seiten 21 bis 24. Die in diesen Literaturstellen beschriebene Partial Least Squares Methode kann für die rechnerische Auswertung des erfindungsgemäßen Analysenverfahrens verwendet werden. Es handelt sich bei dieser Methode um eine Berechnung auf der Basis eines Rechenprogramms, das aus der Veränderung von Infrarot-Spektren von Überzugsfilmen mit unterschiedlichen Konzentrationen der Einzelkomponenten erstellt wird. Dabei werden zur "Eichung" Spektren von Filmen aus Überzugsmitteln bekannter Zusammensetzung mit unterschiedlichen Konzentrationen der Einzelkomponenten eingesetzt. Wichtig dabei ist, daß sowohl die Bedingungen für die Herstellung des homogenen Films, als auch die der Aufnahme der Spektren die gleichen wie für die zu analysierenden Proben sind.

Im Folgenden wird ein Beispiel dafür angegeben, wie die Partial Least Squares Methode erfindungsgemäß ausgewertet werden kann. Ausgangspunkt ist die sogenannte Classic Least Squares Calibration (CLS). Diese CLS Methode beruht auf folgender Formel des Gesetzes von Beer:

$$A_i = \sum_{j=1}^{m} k_{ji} c_j$$

A      Absorption

k      Extinktionskoeffizient x Schichtdicke

i      Frequenz

j      Anzahl der Proben

m      Anzahl der Komponenten in jeder Probe

c      Konzentration

Man kann also folgende Gleichungen schreiben:

|  | | Komponente 1 | | Komponente 2 | |
|---|---|---|---|---|---|

$$A_{1i} = k_{1i}C_{11} + k_{2i}C_{12} \quad \text{für ein erstes Gemisch bekannter Konzentration}$$

$$A_{2i} = k_{1i}C_{21} + k_{2i}C_{22} \quad \text{für ein zweites Gemisch bekannter Konzentration}$$

Da es sich hier um Proben bekannter Konzentration handelt und die Absorption A gemessen werden kann, ist es möglich für jede Frequenz so ein Gleichungspaar anzuschreiban. Die beiden unbekannten Größen $k_{1i}$ und $k_{2i}$ können also exakt bestimmt werden. Wiederholt man nun diese Prozedur für jede Frequenz des Spektrums, so ergibt die Auftragung von $k_{1i}$ und $k_{2i}$ die errechneten Spektren der Reinkomponenten.

Die CLS Methode ist eine erfolgreiche und gut untersuchte Variante der quantitativen Auswertung von IR-Spektren. Dennoch beherbergt sie einige Nachteile und Unzulänglichkeiten, die zur Entwicklung der PLS Methode geführt haben. So sollten für eine korrekte CLS-Analyse alle Substanzen, die Spektralbanden innerhalb des untersuchten Bereiches zeigen, bei der Berechnung berücksichtigt werden. Die Bestimmung von ein oder mehreren Komponenten in einer komplizierten Matrix ist also unmöglich oder zumindest mit Fehlern behaftet.

Bei der Partial Least Squares Methode werden die Spektren in das Produkt zweier Matrizen zerlegt:

$$A = TB + E_A$$

dabei ist

T eine m x r Matrix von Werten
B eine r x n Matrix von "loading vectors"
$E_A$ eine Matrix bestehend aus dem Spektralrauschen sowie dem Fehler der sich aus dem verwendeten Modell ergibt

weiter bedeutet

m die Anzahl von Proben die für die Kalibrierung verwendet werden
n die Anzahl der verwendeten Frequenzen
r die Anzahl an spektralen Komponenten die für eine optimale Vorhersage von Werten notwendig sind.

Die Reihen von B werden oft auch als Eigenvektoren bezeichnet. Diese Gleichung ist eine einfache Transformation des Koordinatensystems, das die Intensitäten von n Frequenzen mit den r Intensitäten in dem neu generierten kompletten Spektrum von r Eigenvektoren in Beziehung setzt. So gesehen kann auch die CLS Methode als Faktoranalyse-Modell gesehen werden, in dem die Faktoren gezwungen werden das Beer'sche Gesetz zu befolgen und ein Faktor für jede Komponente vorhanden ist. Die Intensitäten im CLS-Faktoren Modell sind also die Konzentrationen der einzelnen Komponenten und die Koordination des neu generierten kompletten Spektrums entsprechen den errechneten Reinkomponentenspektren. Man geht davon aus, daß die Spektren der Gemische lineare Kombinationen der Reinkomponentenspektren sind.

Die PLS Methode ist aber nicht daran gebunden, daß die Anzahl der Faktoren auch der Anzahl der bekannten chemischen Komponenten entspricht. Nichtlinearitäten in Beer's Gesetz können also durch Systeme wie das PLS Modell ausgeglichen werden. Während die CLS Methode exakt an Beer's Gesetz gebunden ist, wird das jeweilige Modell bei Systemen wie PLS den Spektraldaten angepaßt, um eine optimale Voraussage von Konzentrationen zu ermöglichen.

Wenn die Faktoranalyse der Kalibrierspektren beendet ist, sind also Intensitäten in den neu generierten kompletten Spektren aus Eigenvektoren mit den jeweiligen Konzentrationen der Komponenten folgendermaßen in Beziehung gesetzt:

$$c = T_v + e_c$$

Hier bedeutet:

v der r x l Vektor von Koeffizienten die Intensitäten mit Konzentrationen in Beziehung setzen
T die Matrix von Intensitäten im neu generierten Koordinatensystem
$e_c$ die Fehler aus Spektralrauschen sowie methodische Fehler

Durch die Zerlegung der Spektren mittels der PLS Methode werden also eher abstrakte Spektren generiert, als errechnete Reinkomponentenspektren wie in der CLS Methode. Die PLS Methode wurde so konzipiert, daß sie Faktoren extrahiert, die sowohl mit den Spektraldaten als auch mit den bekannten Konzentrationen der zu analysierenden Komponenten korrelieren. Fehler sowohl bei den Spektral- als auch bei den Konzentrationsdaten werden hier durch spezielle Rechenschritte minimiert. Geht man davon aus, daß Spektralrauschen fehlt und Beer's Gesetz streng befolgt wird, so sind PLS und CLS Methoden äquivalent.

Die Anzahl der für PLS Berechnungen verwendeten Faktoren ergibt sich durch Abwägen folgender Tatsachen:

- mehr Faktoren ergeben bessere Kalibrierung;
- mehr Faktoren erhöhen die Komplexität des Modells.

Es ist also ein Kompromiß zu suchen, der ein möglichst einfaches Modell ergibt, mit dem man möglichst genaue Vorhersagen treffen kann. Gemäß diesem Kompromiß wird die optimale Anzahl an Faktoren durch sogenannte Kontrollproben ermittelt. Das heißt, daß ein Teil der eingegebenen Kalibrierspektren nicht zur Erstellung einer Kalibrierkurve verwendet werden, sondern als Kriterium für die Güte des gewählten Modells benutzt werden. So wird die Anzahl der Faktoren die zur Berechnung herangezogen werden optimiert.

Die Kalibrierung wird nun einer Reihe von Tests wie Korrelationskoeffizient der Kalibrierkurven für jede einzelne Komponente oder Betrachtung der "loadings" unterzogen. Die "loadings" ergeben für jeden einzelnen Faktor zum einen den Prozentsatz an Gesamtspektralinformation sowie den Prozentsatz an Spektralinformation die jeweilige Komponente betreffend. Daraus ist ersichtlich, ob der gewählte Faktor auch in der Lage ist das System ausreichend zu beschreiben. Ein Wert über 85 % für jede einzelne Komponente erscheint als zweckmäßig.

Nun kann man zur Analyse unbekannter Proben schreiten. Hierbei ist wichtig, daß die Bedingungen sowohl bei der Präparation als auch bei der Aufnahme der Spektren immer dieselben sind. Dies gilt für die Kalibrierproben ebenso wie für die unbekannten, zu analysierenden Proben.

Mit dem erfindungsgemäßen Verfahren ist es möglich, Überzugsmittel bei komplexen Harz-, Vernetzer- und Additivmischungen mit bis zu 13 oder mehr Bestandteilen quantitativ zu erfassen. Im allgemeinen können nicht alle Additivarten bestimmt werden. Additive, die bei diesem Verfahren nicht erfaßt werden, können durch Alternativverfahren wie beispielsweise HPLC (High-Performance Liquid Chromatography), GC (Gaschromatographie) oder SFC (Superkritische Flüssigchromatographie) quantifiziert werden. Die PLS-Auswertungen erlauben beispielsweise das Korrigieren von Fehlansätzen, die Kontrolle von Ultrafiltrationskreisläufen in der Overspray-Rückgewinnung beim Wasserlack-Recycling und die Überprüfung von Koagulaten zur entsprechenden Weiterverwendung. Bevorzugt wird das erfindungsgemäße Verfahren angewandt beim Wasserlackrecycling bzw. beim Oversprayrecycling und bei der Wiedergewinnung von wäßrigen Spritzlacken durch Ultrafiltration und/oder Elektrokoagulation, wie beispielsweise in DE-A1-32 27 227, DE-C2-33 32 457, DE-A1-34 28 300, WO 91/09 666, EP-A1-0 460 122, DE-A1-41 38 088 und in den noch nicht veröffentlichten deutschen Patentanmeldungen P 41 33 130.3, P 42 07 383.9, P 42 13 671.7 beschrieben.

Die quantitative Analyse durch Infrarot-Spektroskopie und Auswertung durch die Partial Least Squares Methode kann von praktisch allen Überzugsmittelsystemen durchgeführt werden. Es eignen sich außer wasserverdünnbaren auch lösemittelverdünnbare Überzugsmittel, sowie nicht-wäßrige, lösemittelfreie Systeme, die pulverförmig oder flüssig sein können. Die Überzugsmittel können ein- oder mehrkomponentig sein, sie können vernetzbar oder chemisch trocknend sein. Auch durch energiereiche Strahlung härtende Lacksysteme können nach dem erfindungsgemäßen Verfahren untersucht werden.

**Beispiel**

Die nichtflüchtigen, pigmentfreien Bestandteile eines wasserverdünnbaren Überzugsmittels setzen sich zusammen aus

1,7 % Komponente A (Melaminharz-Vernetzer)
85,1 % Komponente B (selbstvernetzendes Bindemittel)
9,2 % Komponente C (Bindemittel)
4,0 % Komponente D (Weichmacher)
100,00

Das wasserverdünnbare Überzugsmittel wurde ultrafiltriert. Ca. 10 g Retentat der Ultrafiltration mit einem Festkörpergehalt von 29,8 Gew.-% wurden in ein Zentrifugiergläschen gegeben und bei 20000 U/min. 30 Minuten zentrifugiert. Die obere klare Phase wurde mit jeweils dem halben Volumen Tetrahydrofuran und Isopropanol versetzt und durch Abdestillation von Wasser befreit.

Die in organischen Lösemitteln gelöste Probe wurde auf einen Kaliumbromid-Preßling gegossen, so daß der Preßling bedeckt wurde. Die überschüssige Probelösung wurde durch horizontales Zentrifugieren bei 4000 U/min. entfernt.

Der Film auf dem Kaliumbromid-Preßling wurde bei 120°C 20 Minuten eingebrannt.

Vom eingebrannten und vernetzten Film wurde mit einem Nicolet FT-IR Gerät ein Infrarot-Spektrum aufgenommen und mit analog präparierten und vermessenen Proben bekannter Zusammensetzungen nach dem Partial Least-Square Verfahren unter Zuhilfenahme des PLS Quant Computerprogramms ausgewertet.

Die Eichproben wurden durch Mischen von Lösungen der 4 Komponenten in Propanol-2/Tetrahydrofuran in folgendem Eichbereich hergestellt:

| Komponente | minimaler Gehalt | maximaler Gehalt |
|---|---|---|
| A (Melaminharz-Vernetzer) | 0,50 Gew.-% | 4,00 Gew.-% |
| B (selbstvernetzendes Bindemittel) | 80,00 Gew.-% | 95,00 Gew.-% |
| C (Bindemittel) | 5,00 Gew.-% | 13,00 Gew.-% |
| D (Weichmacher) | 0,00 Gew.-% | 5,00 Gew.-% |

Die Eichung wurde mit 40 Kalibrierungsproben in dem oben angegebenen Bereich durchgeführt.

Es wurden folgende Werte erhalten:

0,5 % Komponente A

88,5 % Komponente B

11,0 % Komponente C

0,00 % Komponente D

$\overline{100,00}$

Durch die Ultrafiltration wurde der Anteil an Komponenten A und D im Retentat reduziert. In der lacktechnischen Prüfung des Retentats zeigte sich, daß der eingebrannte Film geringere Elastizität und geringere Härte aufwies und spröde war. Nach berechneter Zugabe der fehlenden Mengen von A und D unterschied sich der Film lacktechnisch nicht mehr vom Original.

## Patentansprüche

1. Verfahren zur quantitativen Erfassung der nichtflüchtigen, von Pigmenten und Füllstoffen unterschiedlichen Komponenten von Überzugsmitteln oder Überzugsmittelbestandteilen, **dadurch gekennzeichnet,** daß man die Überzugsmittel oder Überzugsmittelbestandteile in flüssiger, pigmentfreier Form auf eine für die Infrarot-Spektroskopie geeignete transparente Unterlage aufbringt und auf dieser, unter zumindest teilweiser Entfernung der flüchtigen Anteile, einen homogenen Film bildet, von diesem Film ein Infrarot-Spektrum erstellt und aus diesem die Menge der interessierenden Komponenten an Hand der Partial Least Squares Methode, unter Auswertung der Infrarot-Spektren von unter identischen Bedingungen aus Überzugsmitteln bekannter quantitativer Zusammensetzung mit unterschiedlichen Konzentrationen der Komponenten, erstellten Filmen, berechnet.

2. Verfahren nach Anspruch 1, dadurch gekennzeichnet, daß aus einem Pigmente und/oder Füllstoffe enthaltenden Überzugsmittel oder Überzugsmittelbestandteil die Pigmente und/oder Füllstoffe durch Zentrifugieren vor der Erstellung des homogenen Films entfernt werden.

3. Verfahren nach Anspruch 1 oder 2, dadurch gekennzeichnet, daß der homogene Film unter Erwärmen ausgebildet wird.

4. Verfahren nach Anspruch 1, 2 oder 3, dadurch gekennzeichnet, daß der homogene Film unter vollständiger Entfernung flüchtiger Bestandteile ausgebildet wird.

5. Verfahren nach einem der vorhergehenden Ansprüche, dadurch gekennzeichnet, daß als für die Infrarot-Spektroskopie geeignete transparente Unterlage ein Kaliumbromid-Preßling oder ein Zinksulfid-Fenster verwendet wird.

6. Verfahren nach Anspruch 5, dadurch gekennzeichnet, daß aus einem wäßrigen Überzugsmittel oder wäßrigen Überzugsmittelbestandteil das Wasser vor dem Auftrag auf einen Kaliumbromid-Preßling als transparente Unterlage, durch ein oder mehrere organische Lösemittel ersetzt wird.

Europäisches
Patentamt

**EUROPÄISCHER RECHERCHENBERICHT**

Nummer der Anmeldung

EP 94 11 2905

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | FARBE + LACK, Bd.89, Nr.6, 1. Juni 1983 Seiten 425 - 426 REBHAN ET AL. 'IDENTIFIZIERUNG DES LACKBINDEMITTELS,USW.' INSGESAMT --- | 1,5 | G01N21/35 G01N33/32 |
| A | PROGRESS IN ORGANIC COATINGS, Bd.13, 1. Dezember 1985 Seiten 253 - 271 HVILSTED 'ANALYSIS OF EMULSION PAINTS' SEITEN 256-261 --- | 1 | |
| A,D | JOURNAL OF CHEMICAL INFORM.AND COMPUTER SCIENCE, Bd.24, Nr.1, 1. Januar 1984 Seiten 20 - 24 FRANK ET AL. 'PREDICTION OF PRODUCT QUALITY,USW.' --- | 1 | |
| A | APPLIED SPECTROSCOPY, Bd.42, Nr.2, 1. Februar 1988 Seiten 217 - 227 FULLER ET AL. 'PARTIAL LEAST SQUARES QUANTITATIVE ANALYSIS,USW.' INTRODUCTION ----- | 1 | RECHERCHIERTE SACHGEBIETE (Int.Cl.6) G01N B05B |

Der vorliegende Recherchenbericht wurde für alle Patentansprüche erstellt

| Recherchenort | Abschlußdatum der Recherche | Prüfer |
|---|---|---|
| DEN HAAG | 9. Dezember 1994 | Boehm, C |

EPO FORM 1503 03.82 (P04C03)